# FASCICULE DE BREVET EUROPEEN

(11) **EP 3 133 935 B1**
(45) Date de publication et mention de la délivrance du brevet: **03.04.2019**
(21) Numéro de dépôt: 15715784.3
(22) Date de dépôt: 25.02.2015
(51) Int. Cl.: A23K 20/10, A61K 36/539, A61K 31/00, A61K 31/352, A61K 31/7048, A23K 20/00, A23K 20/121, A23K 50/10, A23K 50/75, A23K 50/30, A23K 50/80

(54) **UTILISATION D'UN EXTRAIT DE SCUTELLARIA BAICALENSIS COMPRENANT DE LA BAÏCALINE PENDANT LA PÉRIODE DE MISE BAS POUR STIMULER LA LACTATION CHEZ DES ANIMAUX D'ÉLEVAGE.**
VERWENDUNG EINES BAICALIN-HALTIGEN EXTRAKTES VON SCUTELLARIA BAICALENSIS ZUR STIMULIERUNG DER LAKTATION IN NUTZTIEREN WÄHREND DES GEBÄRZEITRAUMS.
USE OF AN EXTRACT OF SCUTELLARIA BAICALENSIS COMPRISING BAICALINE DURING THE PARTURITION PERIOD FOR STIMULATING LACTATION IN LIVESTOCK ANIMALS.

(30) Priorité: 25.02.2014 FR 1451501
(43) Date de publication de la demande: 01.03.2017
(73) Titulaire: Deltavit, 35150 Janze (FR)
(72) Inventeur: ROBERT, Fabrice, F-35580 Guichen (FR)
(74) Mandataire: IP Trust
(86) Numéro de dépôt international: PCT/FR2015/050450
(87) Numéro de publication internationale: WO 2015/128580

(56) Documents cités:
- CN-A- 101 167 529
- CN-A- 101 491 295
- CN-A- 101 971 933
- CN-A- 102 599 380
- CN-A- 102 934 757

## Description

### Domaine de l'invention

Le domaine technique de la présente invention est celui des additifs alimentaires et des aliments pour animaux d'élevage. La présente invention porte sur l'utilisation pendant la période de mise bas d'un extrait de Scutellaria baicalensis pour stimuler la lactation chez des animaux d'élevage choisis parmi la vache et la truie, caractérisée en ce que ledit extrait de Scutellaria baicalensis comprend une quantité connue de baïcaline, et en ce que la dose de baïcaline distribuée aux animaux est comprise entre 0.1 et 5 mg/kg de poids vif et par jour. L'invention est telle que définie dans les revendications.

Les situations d'inconfort d'élevage génèrent des stress et sont corrélées à des altérations physiologiques et comportementales qui sensibilisent les animaux d'élevage aux pathologies. Ces situations de stress ont un impact encore plus négatif sur leur bien être. Une des conséquences est une chute de la productivité de ces animaux fragilisés, que ce soit la production laitière, d'oeufs, de viande ou de tout autre produit. Les phases d'inconfort et de stress se caractérisent également par une production en dessous du potentiel génétique des animaux.

Des améliorateurs de performances ont été utilisés et incorporés dans les aliments, notamment des antibiotiques qui avaient pour but, chez les ruminants par exemple, d'orienter la fermentation ruminale en sélectionnant uniquement les bactéries bénéfiques.

Ces améliorateurs antibiotiques stimulent également la production des animaux d'élevage en réduisant l'inflammation (Niewold, 2007). En effet, les phénomènes inflammatoires réduisent la production de viande, de lait et d'oeufs, (Klasing et al, 1997; Trevisi et al, 2010) lors d'évènements générant des situations d'inconfort ou de stress comme la mise bas, le sevrage, les changements thermiques ou environnementaux, des conditions inadaptées...

Par ailleurs, l'utilisation d'antibiotiques par les éleveurs suit la survenue de situation d'inconfort ou de stress.

Ces améliorateurs antibiotiques représentent de potentiels risques de santé publique par le développement de résistances chez les bactéries et un rejet de la part des consommateurs.

De nombreux extraits végétaux montrent des effets anti-inflammatoires et anti-oxydants (Kim et al, 2004; Rice-Evans et al, 1996). Ils représentent des alternatives nutritionnelles naturelles capables d'agir simultanément sur le bien être des animaux d'élevage et le maintien de leur production quelque soit les conditions d'élevage. Ces extraits végétaux peuvent être intégrés aux aliments des animaux à des suppléments nutritionnels ou à leur eau de boisson.

### Etat de la technique

La demande de brevet CN102805271 divulgue un additif alimentaire anti-stress, anti-diarrhéique, pro-croissance, pour porc sevré issu de la médecine chinoise par les plantes. Cet additif est obtenu par mélange de fins broyats de *Astragalus mongholicus, Scutellaria baicalensis,* du malt, de la réglisse, du dangshen, *Poria cocos* et du rhizome d'Atractylode. Selon la médecine chinoise par les plantes, l'additif selon la présente invention peut accroître les performances de croissance des porcs sevrés, l'additif a des effets anti-diarrhéiques ; le développement du tractus gastro-intestinal des porcs sevrés peut être amélioré ; l'activité des enzymes digestives du tractus gastro-intestinal peut être améliorée pour soutenir le développement du système enzymatique digestif des porcs ; l'environnement micro-écologique du tractus digestif des porcs peut être régulé et stabilisé pour accélérer la croissance des porcs ; la fonction anti-oxydante de l'organisme des porcs sevrés peut être améliorée pour renforcer les capacités compensatrices de l'organisme des porcs face aux irradiations de l'environnement ; la forme de la muqueuse intestinale est améliorée pour éviter l'affaissement des villosités intestinales et renforcer la restauration du tractus intestinal ; et la fonction immunologique de la muqueuse intestinale des porcs sevrés peut être régulée.

La demande de brevet CN103315145 présente un aliment pour porcelets sevrés comprenant un additif composé d'igname de Chine, le perilla pourpre, *Scutellaria baicalensis,* de carthame des teinturiers, de *Houttuynia cordata,* de Leonurus, de Radix dichroa et de réglisse. L'aliment pour porcelets peut fournir les substances nutritives de base une fois les porcelets sevrés. Dans le même temps, par l'addition de l'additif, la croissance et l'immunité des porcelets peuvent être accrues.

La demande de brevet CN101816382 décrit un additif alimentaire et son utilisation dans la résistance aux maladies et pour augmenter la ponte. Cet additif comprend un mélange d'extraits de plantes connues de la médicine traditionnelle chinoise dont un extrait de *Scutellaria baicalensis.*

Le brevet CN101971933 décrit un additif nutritionnel prénatal et un additif nutritionnel post-partum comprenant un mélange de diverses plantes de la médecine traditionnelle chinoise dont *Scutellaria.* Ces deux additifs sont administrés aux vaches avant et après le vêlage. Ces additifs périnatals permettent d'améliorer l'immunité post-partum, leur santé et leur production de lait.

*Scutellaria baicalensis* est une plante d'origine asiatique. Ses extraits comprennent notamment deux principaux principes actifs de la famille des flavones, la baïcaline et la baïcaléine. Ces derniers montrent des activités anti-inflammatoires (Chou et al, 2003; Hsieh et al, 2007) et antivirales.

Littérature non brevet citée :
- Ballou, M.A., Immune responses of Holstein and Jersey calves during the preweaning and immediate postweaned periods when fed varying planes of milk replacer, J. Dairy Sci, 2012;95(12):7319-30
- Chou, T.-C., Chang, L.-P., Li, C.-Y., Wong, C.-S., & Yang, S.-P. (2003). The Antiinflammatory and Analgesic Effects of Baicalin in Carrageenan-Evoked Thermal Hyperalgesia. Anesthesia & Analgesia, 1724-1729. doi:10.1213/01.ANE.0000087066.71572.3F
- Farney, J.K., Mamedova, L.K., Coetzee, J.F., Minton, J.E., Hollis, L.C., Bradford, B.J., Sodium salicylate treatment in early lactation increases whole-lactation milk and milk fat yield in mature dairy cows, J. Dairy Sci, 2013;96(12):7709-18
- Hsieh, C.-J., Hall, K., Ha, T., Li, C., Krishnaswamy, G., & Chi, D. S. (2007). Baicalein inhibits IL-lbeta- and TNF-alpha-induced inflammatory cytokine production from human mast cells via regulation of the NF-kappaB pathway. Clinical and molecular allergy : CMA, 5, 5. doi:10.1186/1476-7961-5-5
- Kim, H. P., Son, K. H., Chang, H. W., & Kang, S. S. (2004). Critical Review Anti-inflammatory Plant Flavonoids and Cellular Action Mechanisms. Journal of Pharmacological Sciences, 245, 229 - 245.
- Klasing, K. C., Korver, D. R., & Korver, I. (1997). Leukocytic Cytokines Regulate Growth Rate and Composition Following Activation of the Immune System Leukocytic Cytokines Regulate Growth Rate and Composition Following Activation of the Immune System 1. Journal of Animal Science, 75, 58-67.
- Niewold, T. a. (2007). The nonantibiotic anti-inflammatory effect of antimicrobial growth promoters, the real mode of action? A hypothesis. Poultry science, 86(4), 605-9.
- Rice-Evans, C. A., Miller, N. J., & Paganga, G. (1996). Structure-antioxidant activity relationships of flavonoids and phenolic acids. Free radical biology and medicine, 20(7), 933-956.
- Sordillo, L.M., Raphael, W., Significance of metabolic stress, lipid mobilization, and inflammation on transition cow disorders, Vet Clin North Am Food Anim Pract, 2013;29(2):267-78
- Trevisi, E., Zecconi, A., Bertoni, G., & Piccinini, R. (2010). Blood and milk immune and inflammatory profiles in periparturient dairy cows showing a different liver activity index. The Journal of dairy research, 77(3), 310-7. doi:10.1017/S0022029910000178

### Inconvénients de l'art antérieur

Les solutions de l'art antérieur sont des associations complexes de différents extraits de plantes nécessitant des dosages fins. Ces associations et leurs dosages sont destinés à une espèce bien spécifique à un stade de développement donné, par exemple les porcelets juste sevrés.

Les solutions de l'art antérieur ne sont pas immédiatement applicables à n'importe quelle espèce de rente. De plus, ces solutions consistent en des extraits bruts de plantes. Or les extraits sont variables dans leur composition en principes actifs et on ne peut pas assurer dans ces conditions une constance dans les effets. Cette variabilité peut même entraîner une inefficacité. La variabilité selon les différentes préparations d'extraits et de mélange d'extraits est très importante et pose un souci de reproductibilité et de fiabilité. De plus, les mélanges de l'art antérieur étant très complexes, il n'est pas possible de connaître l'effet de tel ou tel extrait. Or, il est nécessaire afin d'assurer une constance d'effet d'avoir identifié l'extrait responsable de l'activité biologique recherchée, de pouvoir doser un ou plusieurs principes actifs caractérisant l'extrait et les effets associés et d'assurer un apport constant aux animaux, correspondant à la dose active.

### Solution apportée par l'invention

La présente invention se propose dans son acceptation la plus générale de remédier aux inconvénients de l'art antérieur en proposant l'utilisation pendant la période de mise bas d'un extrait de Scutellaria baicalensis pour stimuler la lactation chez des animaux d'élevage choisis parmi la vache et la truie, caractérisée en ce que ledit extrait de Scutellaria baicalensis comprend une quantité connue de baïcaline, et en ce que la dose de baïcaline distribuée aux animaux est comprise entre 0.1 et 5 mg/kg de poids vif et par jour.

La baïcaline est apportée par un extrait de *Scutellaria baicalensis.*

De façon surprenante, la demanderesse a observé que des extraits, notamment de racine, de *Scutellaria baicalensis* incorporés dans l'alimentation d'animaux de rente augmentait leur production et normalisait les désordres causés par les situations de stress sans avoir recours à toute forme de médication, notamment antibiotique. L'extrait de racine de *Scutellaria baicalensis* comprend en particulier comme principes actifs de la baïcaline et de la baïcaléine. Ces molécules sont traçables tout au long de la fabrication des adjuvants et des aliments décrits ici, ce qui permet de contrôler très finement la dose administrée et ingérée par l'animal au cours de son alimentation.

L'on comprend que la baïcaline est obtenue par extraction selon les méthodes connues de l'Homme du métier.

Préférentiellement, la baïcaline est apportée par un extrait de racine de *Scutellaria baicalensis.*

L'extrait de *Scutellaria baicalensis* est préférentiellement un extrait de la racine de la plante, sous forme de poudre et obtenu par extraction hydro-alcoolique. L'on comprend que l'extrait de *Scutellaria baicalensis* peut être sous forme sèche ou liquide. L'extrait comprend une quantité connue en poids de principe actif consistant en de la baïcaline, classiquement entre 10 et 50% en poids de baïcaline.

La baïcaline est distribuée à l'animal à une dose comprise entre 0,1 et 5 mg/kg, encore plus préférentiellement entre 0,1 et 2 mg/kg de poids vif et par jour.

Il est également décrit ici un adjuvant obtenu par dilution de l'extrait sec de *Scutellaria baicalensis* sur un support, ledit support pouvant être liquide si sa destination est une boisson ou ledit support pouvant être solide si sa destination est un produit d'alimentation solide. Si la destination finale est d'être mélangée à l'eau de boisson des animaux de rente, alors le support de dilution peut être de l'eau ou un mélange d'eau additionné de sels minéraux et/ou d'oligoéléments ou tout autre additif ou solvant connus de l'Homme du métier comme étant classiquement incorporés dans l'eau de boisson. Si la destination finale est un aliment solide, le support peut comporter une céréale ou un mélange de céréales telles que le blé, l'orge, l'avoine ou toute autre matière première ou additifs, solvants, sels minéraux et/ou oligoéléments connus de l'Homme du métier.

L'on entend par situation d'inconfort ou de stress l'ensemble des conditions environnementales ayant un impact négatif sur la production des animaux de rente. Il s'agit des stimuli bien connus de l'Homme du métier impactant la physiologie et le comportement des animaux, comme non limitativement les variations de température, d'humidité, de luminosité, la proximité avec d'autres animaux, la mise bas, le sevrage, le transport, le contact avec l'Homme, les changements de milieu...

L'utilisation selon l'invention est également caractérisée en ce qu'elle ne comprend pas d'antibiotiques.

### Description

Des essais expérimentaux *in vivo* ont été réalisés chez différentes espèces d'animaux de rente soumis à un stress.
La figure 1 présente les résultats de l'évolution de la production laitière journalière moyenne chez la vache recevant un aliment conforme à l'invention.
La figure 2 présente la dose de baïcaline ingérée par les truies au cours de l'essai 2.
La figure 3 présente l'effet de la dose journalière de baïcaline sur la consommation alimentaire pendant la phase d'inconfort thermique au cours de l'essai 4.

Il a été démontré que les situations d'inconfort induites par la mise bas sont susceptibles d'altérer l'état de santé et la lactation (Ballou, 2012; Farney et al., 2013; Sordillo & Raphael, 2013).

### Essai 1 : étude de l'effet de l'administration d'un aliment comprenant l'adjuvant comportant un extrait de Scutellaria baicalensis sur un support solide sur la production laitière par des vaches laitières soumises à un stress

### Protocole :

L'essai a été réalisé sur un troupeau de cent vaches laitières de race Prim'Holstein.

24 vaches laitières vêlant dans la même période ont été réparties en deux groupes de douze selon la date de vêlage, le rang de lactation, la quantité de lait produit, le taux butyreux, le taux protéique à 305 jours de la lactation précédente pour les multipares, l'index lait pour les primipares.

La ration à base d'ensilage de maïs est identique pour les deux lots à l'exception de l'ajout d'un adjuvant conforme à l'invention incluant 10% d'un extrait de *Scutellaria baïcalensis* sur un support carbonate de calcium. Cet adjuvant est incorporé à 0.5% dans l'aliment de production (17% protéine brute, 1 UFL) distribué à raison de 2 kg par vaches et par jour dans le lot essai. Chaque vache reçoit donc 1 gramme par jour d'extrait de *Scutellaria baïcalensis* soit 333 mg de baïcaline, soit 0.5 mg/kg de poids vif.

### Résultats sur la production laitière :

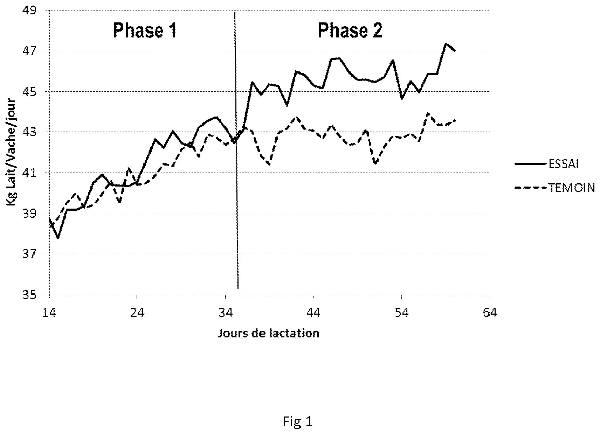

La production laitière est significativement améliorée sur les deux mois de suivi. L'effet de l'extrait de *Scutellaria baïcalensis* sur la lactation s'exerce après le premier mois (phase 2 sur la figure 1). La figure 1 montre l'amélioration de la production laitière journalière moyenne chez des vaches recevant un aliment supplémenté en extrait de *Scutellaria baicalensis* conformément à l'invention par rapport aux vaches du groupe témoin. Le tableau 1 résume les moyennes de production laitière des vaches selon leur alimentation. La production laitière est significativement augmentée chez les vaches du groupe recevant un aliment supplémenté en *Scutellaria baïcalensis.*

**Tableau 1. Production laitière en kg / vache / jour (ns : non significatif)**

| **Lot** | **Total** | **Période 1** | **Période 2** |
|---|---|---|---|
| **Scutellaria** | 44 | 42.8 | 45.9 |
| **Témoin** | 42.5 | 41.7 | 42.9 |
| ***p*** | *<0.05* | *ns* | *<0.05* |

### Essai 2 : Etude de l'effet d'un aliment lactation comprenant un extrait de Scutellaria baïcalensis sur la lactation des truies.

Contrairement aux vaches laitières dont la production laitière est directement mesurable, la mesure de la production laitière des truies est indirecte et se mesure par le gain de poids des porcelets allaités par leur mère (GMQ - Gain Moyen Quotidien en grammes).

### Protocole :

55 truies sont réparties en deux groupes. Les aliments dont les caractéristiques figurent dans le tableau 2 sont distribués pendant toute la lactation des truies. L'adjuvant *Scutellaria baïcalensis* est composé d'un extrait de *Scutellaria baïcalensis* incorporé à 1.5% sur un support. L'aliment ESSAI contient 150 mg de l'extrait de *Scutellaria baïcalensis* par kg soit 50 mg par kg d'aliment de baïcaline. Ces aliments sont distribués aux truies, trois jours avant mises bas jusqu'au sevrage des porcelets (21 jours après mise bas). 632 porcelets sont intégrés à l'essai.

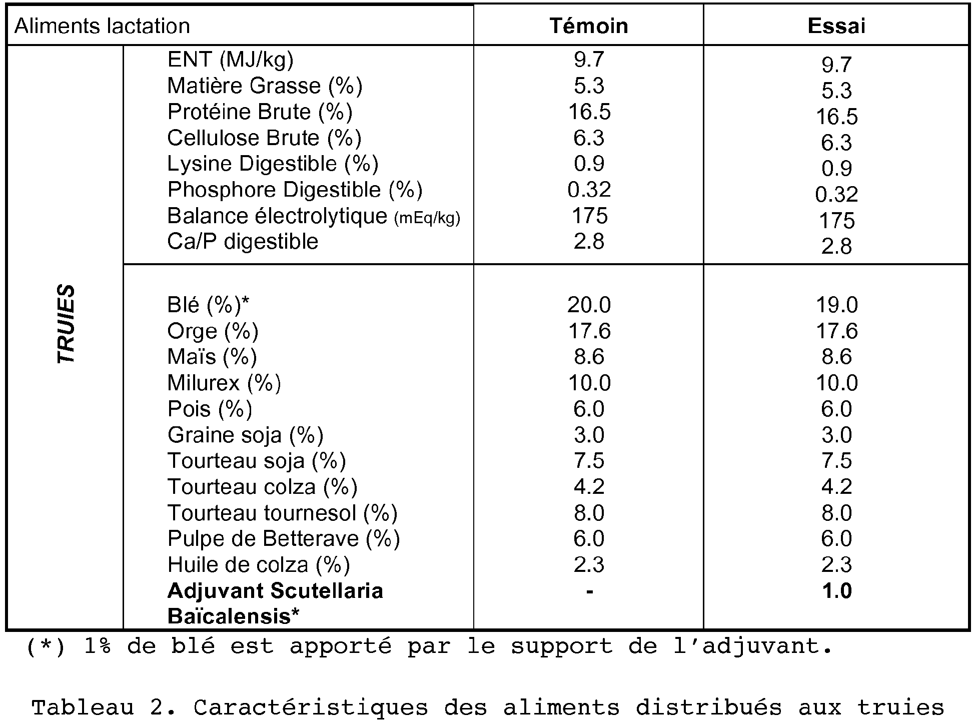

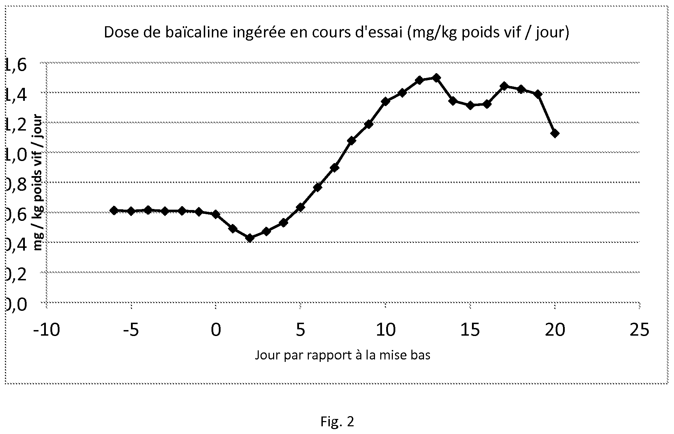

La consommation d'aliment de chaque truie est enregistrée et permet de calculer la dose journalière ingérée par kg de poids vif et par jour au cours de l'essai. Cette dose journalière est représentée en figure 2.

### Résultats de l'essai 2 :

L'aliment ESSAI ne diffère de l'aliment témoin que par l'incorporation de l'adjuvant conforme à l'invention comportant un extrait de *Scutellaria baicalensis.*

Les porcelets des truies des deux lots ont des poids équivalents à la naissance.

Le tableau 3 ci-dessous présente les effets d'un extrait de *Scutellaria baïcalensis* sur la production laitière des truies évaluée par la croissance des porcelets allaités (GMQ : gain moyen quotidien des porcelets allaités). Le poids au sevrage et le GMQ sont significativement plus élevés chez les porcelets nourris par une truie ayant consommé un aliment supplémenté en *Scutellaria baicalensis.*

**Tableau 3. Poids moyen des porcelets selon l'alimentation de la truie**

| | Poids moyen des porcelets à la naissance (kg) | Poids au sevrage (kg) | GMQ (g/j) |
|---|---|---|---|
| Témoin | 1.33 | 5.68 | 235 |
| *Scutellaria baïcalensis* | 1.32 | 6.04 | 264 |
| *Comparaison de moyenne - significativité* | *ns* | <*0.001* | <*0.001* |

Les pesées des porcelets révèlent une croissance plus forte des porcelets dont les mères consomment un aliment comportant un extrait de *Scutelaria baïcalensis.* Ceci démontre que la production laitière des truies est stimulée par l'extrait de *Scutellaria baïcalensis.*

### Essai 3 (en dehors de l'invention) : étude de l'effet de l'administration d'un aliment comprenant l'adjuvant à l'extrait de Scutellaria baicalensis sur un support solide sur la consommation alimentaire de poules pondeuses soumises à un stress thermique

### Protocole :

72 poules pondeuses Isabrown âgées de trente semaines au début de l'essai ont été réparties en trois groupes de douze cages de deux poules. L'essai est conduit jusqu'à 38 semaines.

Le groupe 1 reçoit l'aliment témoin dont les caractéristiques figurent dans le tableau 4.

Le groupe 2, autrement appelé groupe OTC, reçoit l'aliment témoin complémenté d'un antibiotique, 400 ppm d'oxytétracycline.

Le groupe 3, autrement appelé groupe SCU, reçoit l'aliment témoin complémenté d'un extrait de racine de *Scutellaria baicalensis* apportant 6mg/kg d'aliment de baïcaline.

**Tableau 4 : formule de l'aliment de base distribué**

| **Nom matières premières** | **%** |
|---|---|
| BLE | 30,00 |
| MAIS | 36,62 |
| Tourteau SOJA | 21,40 |
| HUILE SOJA | 0,80 |
| BICARBONATE de sodium | 0,12 |
| PHOSPHATE BICALCIQUE | 1,26 |
| CARBONATE | 7,54 |
| SEL | 0,28 |
| METHIONINE 15/BLE 85 | 0,98 |
| Complément minéral et vitaminique | 1 |

Après trois semaines de distribution des aliments expérimentaux, les pondeuses sont soumises à une variation de température. La température de l'environnement est augmentée de 22°C (température de confort thermique) à 35°C pendant 5 jours. Consommation d'aliment, nombre d'oeufs pondus et poids moyen des oeufs sont enregistrés toutes les semaines et tous les jours pendant la semaine de température élevée et la semaine suivante.

L'essai a été découpé en trois phases : phase pré-augmentation thermique, phase de stress thermique et phase post-température élevée.

### Résultats :

Les consommations pendant la phase pré-augmentation thermique sont identiques entre les différents groupes. Pendant la phase de stress thermique, les consommations journalières par poule sont respectivement de 50.9 g, 53.5 g et 59.7 g pour les groupes témoin, OTC et SCU, le groupe SCU montrant une consommation significativement supérieure (p<0.05). Les poules pondeuses du groupe 3, SCU ont augmenté leur consommation alimentaire de 17% lors de la phase de stress thermique. Le taux de ponte sur les 2 semaines intégrant la phase de stress thermique et la semaine suivante sont respectivement de 85.2, 88.5 et 87.2 % pour les poules recevant l'aliment témoin, l'aliment supplémenté avec l'antibiotique et l'extrait de *Scutellaria baïcalensis.*

### Essai 4 (en dehors de l'invention) : étude de l'effet de l'administration d'un aliment comprenant l'adjuvant constitué d'un extrait de Scutellaria baicalensis sur un support solide sur la croissance de poulets soumis à une température élevée

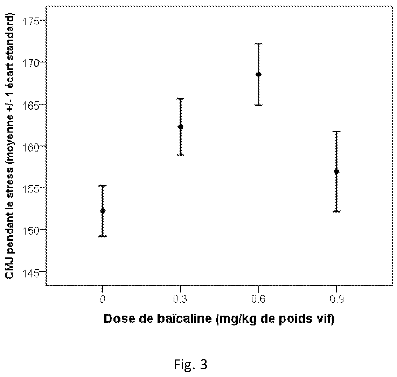

160 poulets Ross PM3 jaune sont répartis en quatre groupes de 20 cages de deux poulets. Le groupe 1 est le groupe témoin. Les poulets du groupe 1 reçoivent un aliment témoin dont la composition est récapitulée en tableau 5. Le groupe 2 reçoit l'aliment témoin additionné d'un adjuvant conforme à l'invention comportant un extrait de *Scutellaria baicalensis* à 3 mg/kg d'aliment de baïcaline, le groupe 3 reçoit le même aliment avec 6 mg/kg de baïcaline et le groupe 4, 9 mg/kg d'aliment. Compte tenu de la quantité d'aliment consommé la dose moyenne de baïcaline ingérée par kg de poids vif et par jour a pu être calculée (tableau 6, figure 3). L'extrait de *Scutellaria baïcalensis* est apporté par un adjuvant contenant 0.5% de l'extrait et 99.5% de blé en substitution du blé dans la formule.

**Tableau 5. Composition de l'aliment de base distribué**

| **INTITULE** | **%** |
|---|---|
| BLE | 30,00 |
| MAIS | 34,25 |
| Graines SOJA | 4,20 |
| Tourteaux SOJA | 25,70 |
| HUILE SOJA | 1,50 |
| BICARBONATE de SODIUM | 0,21 |
| PHOSPHATE BICALCIQUE | 1,43 |
| CARBONATE | 0,62 |
| SEL | 0,20 |
| METHIONINE 15 / BLE 85 | 1,07 |
| L-LYSINE 20 / BLE 75 | 0,42 |
| PREMIX Oligo vitaminique | 0,40 |

Du vingt-cinquième au trentième jour, les poulets sont soumis à une augmentation artificielle de la température de l'environnement de 22°C à 36°C afin d'induire un stress thermique.

Les poulets sont pesés avant et après la phase de stress thermique et leur consommation alimentaire est mesurée (jour 24 et jour 30). L'essai se prolonge jusqu'à 30 jours.

### Résultats :

Le tableau 6 résume les résultats obtenus chez les quatre groupes de poulets pendant la phase de stress thermique (jour 24 à jour 30).

**Tableau 6. Poids des poulets selon leur alimentation**

| Groupe | Dose (mg/kg de poids vif / jour) | Poids initial avant la phase de stress thermique (g) | Poids final après la phase de **stress** thermique (g) | GMQ (g/j) | CMJ (g/j) | ICT |
|---|---|---|---|---|---|---|
| Témoin | 0 | 1290 | 1786 | 82.6 | 152.2 | 1.94 |
| Baïcaline | 0.3 | 1305 | 1852 | 91.1 | 162.3 | 1.80 |
| Baïcaline | 0.6 | 1257 | 1837 | 96.6 | 168.5 | 1.74 |
| Baïcaline | 0.9 | 1288 | 1804 | 86.0 | 157.0 | 1.94 |
| *Comparaison de moyenne - significativité (poids initial en covariable)* | | *ns* | <*0.01* | *0.05* | <*0.01* | *0.06* |

| | | | | | | |
|---|---|---|---|---|---|---|
| GMQ, gain moyen quotidien - CMJ, consommation moyenne journalière - ICT, indice de consommation technique | | | | | | |

Avant la période de stress lié à une température excessive dans les bâtiments, la croissance des oiseaux recevant les deux aliments est équivalente (poids à 24 jours non différents entre les lots). La consommation alimentaire moyenne journalière (CMJ) du groupe recevant l'aliment comprenant un extrait de *Scutellaria baicalensis* à hauteur de 0,6 mg/kg de poids vif et par jour a été plus importante de 10,7% et le gain moyen quotidien (GMQ) a été plus important de 17% par rapport au groupe témoin pendant la phase de stress thermique. Cela entraîne une meilleure croissance des poulets traités par rapport aux poulets du groupe témoin. La dose de 0,6 mg de baïcaline par kg de poids vif et par jour est la dose pour laquelle les effets sont les plus intéressants (figure 3).

### Essai 5 (en dehors de l'invention) : étude de l'effet de l'administration d'un aliment comprenant l'adjuvant à l'extrait de Scutellaria baicalensis sur un support solide sur l'amélioration de l'état de santé et sur la résistance aux pathologies de porcelets soumis à un stress

Les porcelets sont âgés de 42 jours au début de l'essai. Trois groupes sont constitués : un groupe témoin, un groupe recevant le même aliment complémenté avec 100 ppm d'un antibiotique (tylosine) et enfin le dernier groupe reçoit un aliment complémenté de 100 ppm d'un extrait de *Scutellaria baïcalensis,* soit 30 ppm de baïcaline par kg d'aliment. Les pathologies sont enregistrées ainsi que les traitements curatifs réalisés dans chaque groupe. Les résultats figurent dans le tableau 7.

| | **Témoin** | **Antibiotique Tylosine (100 ppm dans l'aliment)** | **Extrait de *Scutellaria baïcalensis* (100ppm dans l'aliment)** | ***P (Chi²)*** |
|---|---|---|---|---|
| Porcs mis en essai | 19 | 25 | 20 | |
| Nombre de morts | 0 | 0 | 0 | |
| Retirés en cours d'essai | 0 | 1 | 0 | |
| Mortalité+élimination (%) | 0,0 | 4,0 | 0 | |
| | | | | |

| **Nombre de traitements** | **15** | **14** | **5** | **<*0,05*** |
|---|---|---|---|---|
| % traitements | 78,9 | 56,0 | 25 | |
| Diarrhées aqueuses | 7 | 4 | 3 | |
| Maigre | 0 | 2 | 0 | |
| Boiterie | 0 | 0 | 0 | |
| Toux | 8 | 7 | 2 | |
| Symptômes nerveux | 0 | 1 | 0 | |
| | | | | |

| **Nombre Porcs traités** | **13** | **13** | **5** | ***<0,05*** |
|---|---|---|---|---|
| % Porcs traités | 68,4 | 52 | 25 | |
| % Porcs non traités | 31,6 | 48 | 75 | |

| Porcs traités par cause | | | | |
|---|---|---|---|---|
| Diarrhées aqueuses | 7 | 4 | 3 | |
| Maigre | 0 | 2 | 0 | |
| Boiterie | 0 | 0 | 0 | |
| Toux | 8 | 7 | 2 | |
| Symptômes nerveux | 0 | 1 | 0 | |

| | | | | |
|---|---|---|---|---|
| Tableau 7. Episodes pathologiques chez des porcelets sevrés selon leur alimentation. | | | | |

On observe significativement moins de malades dans le groupe recevant dans l'aliment un adjuvant comportant conformément à l'invention un extrait de *Scutellaria baïcalensis* et le nombre de traitements curatifs est également réduit. La typologie des symptômes enregistrés montre une amélioration générale non spécifique de l'état de santé car les problèmes digestifs comme les problèmes respiratoires sont améliorés.

## Revendications

1. Utilisation pendant la période de mise bas d'un extrait de *Scutellaria baicalensis* pour stimuler la lactation chez des animaux d'élevage choisis parmi la vache et la truie, **caractérisée en ce que** ledit extrait de *Scutellaria baicalensis* comprend une quantité connue de baïcaline, et **en ce que** la dose de baïcaline distribuée aux animaux est comprise entre 0,1 et 5mg/kg de poids vif et par jour.

2. Utilisation selon la revendication 1, **caractérisée en ce que** la dose de baïcaline est comprise entre 0,1 et 2 mg par kg de poids vif et par jour.

3. Utilisation selon l'une des revendications précédentes, **caractérisée en ce que** ladite stimulation est obtenue sans utilisation d'antibiotique.

## Patentansprüche

1. Verwendung eines Extrakts von Scutellaria baicalensis während des Werfens zur Stimulierung der Laktation bei Zuchttieren, die ausgewählt werden aus Kühen und Säuen, **dadurch gekennzeichnet, dass** besagtes Scutellaria baicalensis-Extrakt eine bekannte Menge an Baicalin umfasst, und dadurch, dass die an die Tiere ausgegebene Menge an Baicalin bei täglich 0,1 bis 5 mg/kg Lebendgewicht liegt.

2. Verwendung nach Anspruch 1, **dadurch gekennzeichnet, dass** die Baicalin-Dosis bei täglich 0,1 bis 2 mg pro kg Lebendgewicht liegt.

3. Verwendung nach einem der vorhergehenden Ansprüche, **dadurch gekennzeichnet, dass** besagte Stimulation ohne Verwendung von Antibiotika erzielt wird.

## Claims

1. Use during the calving/farrowing period of an extract of *Scutellaria baicalensis* to stimulate lactation in farm animals selected from cows and sows, **characterised in that** said extract of *Scutellaria baicalensis* comprises a known amount of baicalin, and **in that** the dose of baicalin dispensed to the animals is between 0.1 and 5mg/kg of live weight per day.

2. Use according to claim 1, **characterised in that** the dose of baicalin is between 0.1 and 2 mg per kg of live weight per day.

3. Use according to any of the preceding claims, **characterised in that** said stimulation is obtained without the use of antibiotics.
